# FASCICULE DE BREVET EUROPEEN

(11) **EP 1 116 023 B1**
(45) Date de publication et mention de la délivrance du brevet: **17.04.2002**
(21) Numéro de dépôt: 99943040.8
(22) Date de dépôt: 24.09.1999
(51) Int. Cl.: G01N 33/18

(54) **PROCEDE ET DISPOSITIF POUR REALISER DES PROFILS VERTICAUX DE MESURES DE GRANDEURS CARACTERISTIQUES D'UNE MASSE D'EAU MARINE**
VERFAHREN UND VORRICHTUNG ZUM ERZEUGEN VON VERTIKALEN MESSPROFILEN VON MEERWASSERCHARAKTERISTISCHEN GRÖSSEN
METHOD AND DEVICE FOR PRODUCING VERTICAL PROFILES OF MEASURED VARIABLES CHARACTERISING A BODY OF SEA WATER

(30) Priorité: 25.09.1998 FR 9812037
(43) Date de publication de la demande: 18.07.2001
(73) Titulaire: SOCIETE BRETONNE D'INSTRUMENTATION OCEANOGRAPHIQUE BRIO, 29900 Concarneau (FR)
(72) Inventeur: GUINARD, Jean-Paul, F-29900 Concarneau (FR)
(74) Mandataire: Colas, Jean-Pierre
(86) Numéro de dépôt international: FR9902273
(87) Numéro de publication internationale: WO0019197

(56) Documents cités:
- WO-A-96/29597

## Description

La présente invention est relative à un procédé pour réaliser des profils verticaux de la mesure d'au moins une grandeur caractéristique d'une masse d'eau marine, à l'aide d'une sonde plus légère que l'eau et équipée d'un capteur de mesure de ladite grandeur, procédé du type suivant lequel, a) on coule ladite sonde sur le fond marin par lestage, b) on commande la remontée de la sonde et l'activation du capteur pour l'enregistrement de la mesure de ladite grandeur au long de la colonne d'eau traversée par la sonde jusqu'à la surface de la masse d'eau, et c) on commande une transmission des mesures relevées vers un centre d'exploitation de ces mesures.

La présente invention est aussi relative à un dispositif pour la mise en oeuvre de ce procédé et à une sonde formant partie de ce dispositif.

On connaît un tel procédé de la demande de brevet PCT n° WO 96/29597 du 20 Mars 1996 déposée au nom de BRIO, demande désignée ci-dessous sous le nom de "demande de brevet de référence".

La demande de brevet de référence est relative à un procédé et à un dispositif pour réaliser des profils verticaux de mesures de grandeurs caractéristiques d'une masse d'eau marine et transmettre les données recueillies à un centre de traitement, et plus particulièrement à un tel procédé et à un tel dispositif faisant appel à des sondes plus légères que l'eau parquées sur le fond de la mer, équipées de capteurs de mesure sensibles auxdites grandeurs. Elle a pour but général de proposer un moyen performant, facile à mettre en oeuvre et économique pour réaliser des observations in situ de surveillance opérationnelle de l'évolution des caractéristiques d'une masse d'eau marine, parfois en complément d'autres dispositifs d'observation. Le domaine intéresse la recherche scientifique, la météorologie, la navigation maritime, la défense, la pêche.

La présente invention perfectionne le procédé et le dispositif décrits dans la demande de brevet en référence, de manière à (1) améliorer les performances du dispositif, (2) accroître sa facilité de mise en oeuvre, (3) réduire son prix de revient.

### Arrière-plan de l'invention

Les solutions techniques décrites dans la demande de brevet de référence présentent les limitations suivantes:
(1) un effet de sillage altère les performances de mesure des capteurs: comme ceux-ci sont situés à l'arrière de la sonde, le flux d'eau qui les intéresse est modifié par le déplacement de la sonde,
(2) le liquide de référence contenu dans une membrane souple, est, pendant le parcage au fond, soumis aux hautes pressions du milieu. Or, ses caractéristiques dépendent de la pression, ce qui complique les procédures d'étalonnage des capteurs; surtout, la loi de correspondance qui relie ces caractéristiques à la pression de stockage est mal connue,
(3) la solution technique proposée dans la demande de brevet en référence comporte des difficultés de réalisation industrielle importantes et un coût élevé: - nombreuses pièces, - étanchéités difficiles entre pièce d'antenne et corps de sonde, entre pièce porte-capteurs et ce même corps, entre capsule de confinement et corps de sonde, -mécanisme de séparation relativement complexe,
(4) les dimensions et le poids d'une sonde sont grevés par divers facteurs, en particulier: corps non optimisé pour résister à la pression, pièce d'antenne lourde, utilisation de matériaux de flottabilité à mousse syntactique de densité élevée (>0,55), ce qui réduit les facilités de mise en oeuvre, en particulier par avion.

La présente invention a donc pour but de remédier aux inconvénients identifiés ci-dessus.

On atteint ce but de l'invention, ainsi que d'autres qui apparaîtront dans la suite de la présente description, avec un procédé pour réaliser des profils verticaux de la mesure d'au moins une grandeur caractéristique d'une masse d'eau marine, à l'aide d'une sonde plus légère que l'eau et équipée d'un capteur de mesure de ladite grandeur, du type suivant lequel, a) on coule ladite sonde sur le fond marin par lestage, b) on commande la remontée de la sonde et l'activation du capteur pour l'enregistrement de la mesure de ladite grandeur au long de la colonne d'eau traversée par la sonde jusqu'à la surface de la masse d'eau, et c) on commande une transmission des mesures relevées vers un centre d'exploitation de ces mesures, ce procédé étant remarquable en ce que, à l'étape a), on coule la sonde avec le capteur disposé du côté d'une extrémité de celle-ci qui est orientée vers le fond marin, et en ce que, à l'étape b), on commande un basculement de la sonde, à la remontée de celle-ci, de manière que ledit capteur soit alors tourné vers la surface de l'eau.

Pour la mise en oeuvre de ce procédé, l'invention fournit un dispositif comprenant une sonde plus légère que l'eau et équipée d'au moins un capteur de mesure d'une grandeur caractéristique d'une masse d'eau marine, d'une capsule de protection dudit capteur et d'un lest raccordé à ladite capsule de protection, caractérisé en ce que ladite capsule de protection prend la forme d'une enceinte étanche résistante à la pression hydrostatique, la paroi de ladite enceinte étant équipée d'un bouchon à ouverture commandée électriquement pour obtenir la séparation de la capsule de protection et de la sonde et la remontée subséquente de celle-ci sous l'effet de la poussée d'Archimède qu'elle reçoit.

Suivant l'invention, la sonde formant partie de ce dispositif comprend au moins un capteur d'une grandeur caractéristique d'une masse d'eau marine, des moyens électroniques de traitement d'un signal émis par ledit capteur, une antenne alimentée par lesdits moyens pour émettre des signaux radioélectriques représentatifs dudit signal, et des moyens propres à donner une flottabilité positive à ladite sonde, cette sonde étant remarquable en ce que ledit capteur est porté par un organe allongé traversant un corps creux rigide constituant à la fois lesdits moyens de flottabilité et un moyen de support dudit organe, ledit capteur étant extérieur audit corps creux alors que lesdits moyens électroniques sont intérieurs à ce corps.

D'autres caractéristiques et avantages de la présente invention apparaîtront à la lecture de la description qui va suivre et à l'examen du dessin annexé dans lequel:
la Fig. 1 est un plan en coupe d'un ensemble sonde-capsule de protection, faisant partie du dispositif suivant l'invention,
la Fig.2 est une vue en élévation de ce dispositif, immédiatement après sa mise à l'eau,
les figures 3a et 3b illustrent le fonctionnement de taquets formant partie du dispositif selon l'invention, et
la Fig. 4 représente schématiquement la suite des mouvements d'une sonde selon l'invention, depuis son immersion jusqu'à sa remontée à la surface de l'eau.

### Description de l'invention.

Le dispositif objet de la présente invention se compose d'une sonde, d'une capsule de protection et d'un harnais d'assemblage temporaire de ladite sonde et de ladite capsule.

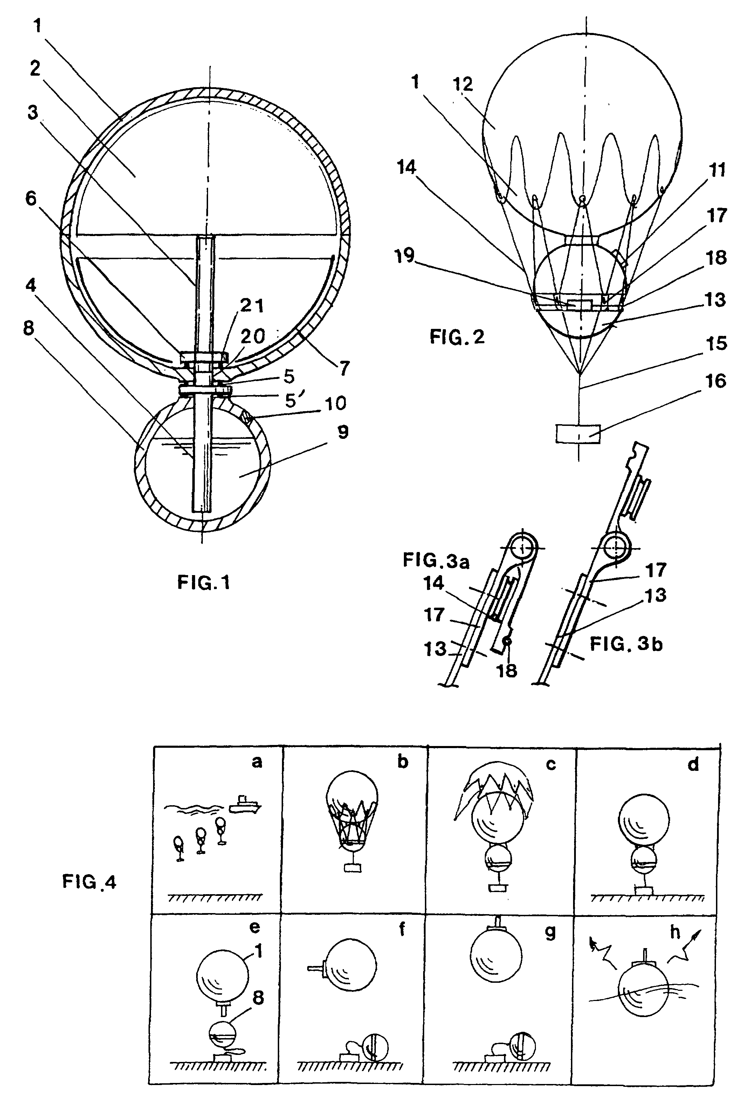

La sonde se compose (Fig. 1): d'un corps 1 contenant des moyens électroniques 2 et d'un organe allongé tel qu'une tige de liaison 3 portant elle-même des capteurs des grandeurs à mesurer (température, salinité, pression hydrostatique, etc...), référencées globalement 4. Une antenne radioélectrique 7 est disposée dans le corps 1 et/ou dans la tige de liaison 3.

Le corps 1 se présente sous la forme d'un réservoir étanche et résistant à la pression de parcage des sondes. De forme sphérique (ou éventuellement ovoïde), il est réalisé en verre borosilicate ou dans une matière isolante et transparente aux ondes radio. Il est constitué par l'assemblage de deux demi-coquilles, selon un procédé classique pour ce type d'application (colle silicone et ruban adhésif). En son centre, une des demi-coquilles comporte un bossage percé d'un trou 20.

Le corps 1 contient des moyens électroniques constitués d'une batterie, d'une horloge, d'un programmateur, d'une mémoire morte, d'une électronique "capteurs", d'un convertisseur analogique/numérique, d'un microprocesseur, d'une mémoire de données, d'un codeur-émetteur radiofréquence raccordé à l'antenne radioélectrique, ces moyens étant conformes à ceux décrits dans la demande de brevet de référence. Ils assurent le traitement des signaux délivrés par les capteurs et leur conversion en signaux radioélectriques émis par une antenne, pour la transmission de ces signaux comme décrit également dans cette demande de brevet.

La tige de liaison 3 est fixée sur le corps 1, à travers un trou 20 traversant sa paroi, par assemblage étanche à vis-écrou serrant un joint d'étanchéité torique 21 : la tige de liaison 3 est filetée sur une partie de sa longueur pour être assemblée par serrage de l'écrou 6 (Fig. 1) sur le corps 1, le passage étant rendu étanche par pressage d'un autre joint torique 5. Les capteurs 4 sont reliés aux moyens électroniques 2 par des conducteurs moulés dans la tige de liaison 3, de façon à réaliser une traversée étanche. Le corps 1 et la tige de liaison 3 servent de structure mécanique pour l'ensemble des organes de la sonde.

Les éléments rayonnants de l'antenne radioélectrique 7 sont disposés dans le corps 1 et/ou la tige de liaison 3 de façon à rayonner dans l'hémisphère défini par les capteurs, selon un diagramme optimisé pour la liaison recherchée. Ces éléments peuvent être réalisés soit sous forme de réseau imprimé sur support hémisphérique disposé autour de la tige de liaison 3 et plaqué à l'intérieur de la demi-sphère supérieure du corps 1, soit sous forme de fouet contenu dans la tige elle-même, complété par un plan de sol disposé à l'équateur du corps 1.

Le dimensionnement du corps 1 (diamètre, épaisseur, matériau) résulte de deux impératifs principaux: (1) la flottabilité de la sonde en eau de mer doit être au moins égale au double de son poids, pour assurer une émersion suffisante en surface pour permettre un rayonnement d'antenne convenable, (2) son diamètre doit permettre de placer une antenne de dimensions aussi proches que possible du quart d'onde, soit 17 cm à 400 Mhz.

La batterie, les organes électroniques et éventuellement un lest sont disposés dans le *corps* 1 de façon à placer centre de gravité et centre de poussée sur l'axe de symétrie de la sonde équipée, le centre de poussée étant situé entre les capteurs et le centre de gravité, de façon à conférer à la sonde libre une grande stabilité hydrostatique, capteurs 4 et antenne 7 vers le haut comme on le verra plus loin, tant en immersion qu'en surface.

Sur la figure 1, il apparaît que l'extrémité de la tige 3 qui sort du corps 1 est enveloppée par une capsule 8 de protection et de confinement.

La capsule 8 est conçue pour assurer l'étanchéité et résister à la pression de parcage au fond. De forme quelconque, sphérique par exemple, elle est réalisée dans un matériau conçu pour assurer une très bonne étanchéité à toute contamination de l'extérieur (eau de mer) vers l'intérieur. Comme le corps 1 de la sonde, elle comporte un bossage percé d'un trou pour le passage de la tige de liaison 3. L'étanchéité entre la capsule 8 et la tige de liaison 3 est assurée par pressage d'un joint torique d'étanchéité 5' entre ces deux organes. Un second orifice, percé selon une radiale à 30° environ de l'axe du premier, reçoit un bouchon 10 à ouverture commandée électriquement par une minuterie 11 (voir Fig. 2). L'ouverture de cet orifice met en équipression l'extérieur et l'intérieur de la capsule 8. Elle est obtenue soit par électrolyse d'un bouchon en Inconel, selon un procédé utilisé couramment pour ce type d'application, soit par ouverture d'un clapet. La capsule 8 est remplie aux deux tiers environ de son volume par un liquide de référence 9, selon une disposition décrite dans la demande de brevet de référence.

Au moment de sa mise à l'eau, le dispositif suivant l'invention comprend (Fig. 2) : une sonde et sa capsule 8 de confinement, un harnais constitué d'une bâche 12 épousant en forme le haut du corps de sonde 1, d'une bâche équipée 13 épousant en forme la capsule 8, et d'un sanglage 14. Un orin 15 est maillé sur la bâche équipée 13 et relié à un corps mort 16. Dans certaines applications, on pourra intégrer un lest dans la capsule de confinement et supprimer l'orin 15, le corps mort 16 et leurs fixations sur la bâche 13.

Sur la bâche équipée 13 sont disposés de place en place des taquets à ressorts 17 (voir Fig. 3a et 3b) dans lesquels passe le sanglage 14. Les taquets 17 sont maintenus fermés (Fig. 3a) par un câble de ceinture 18 tendu et fermé par un largueur à pression 19 (voir Fig. 2). Au montage, le corps de sonde 1 et la capsule de confinement 8 sont tenus l'un contre l'autre par le harnais ainsi constitué et pressés l'un contre l'autre par mise en tension du sanglage 14. L'ensemble du dispositif est mis à l'eau dans cette configuration ; il est conçu pour résister aux chocs de manutention et de mise à l'eau. Etant plus lourd que l'eau, il s'enfonce ; lorsque la pression atteint la valeur de consigne du largueur à pression 19, soit quelques dizaines de mètres, celui-ci libère le câble de ceinture 18, - les taquets 17 s'ouvrent (Fig. 3b) sous l'action de leurs ressorts, - le sanglage 14 est libéré, ainsi que la bâche 12. Ces derniers, plus légers que l'eau, remontent en surface.

Le mouillage d'une base de réalisation de profils verticaux de mesures en un lieu donné est réalisé par mises à l'eau successives de plusieurs dispositifs selon l'invention à partir d'un navire ou d'un aéronef en route (Fig.4a). Chaque station s'enfonce ensuite, sous l'effet de son poids (Fig. 4b). Sous quelques dizaines de mètres d'eau, la bâche 12 se détache du reste du dispositif (Fig. 4c). Ce dernier se pose en position de parcage sur le fond (Fig. 4d). Sur ordre commandé par la minuterie 11, le bouchon 10 s'ouvre et met en communication l'intérieur et l'extérieur de la capsule 8. Aucune force de pression ne s'opposant plus à la poussée hydrostatique exercée sur la sonde, celle-ci s'extrait de la capsule 8 (Fig. 4e) ; l'électronique est activée. Immédiatement après, la sonde se retourne, sous l'effet des forces hydrostatiques et de gravité (Fig. 4f). (Nota : on peut profiter de ce retournement pour décharger le corps de sonde des salissures provenant des hautes couches de l'océan, susceptibles d'altérer la pesée et le Cx de la sonde pendant sa remontée). Elle poursuit ensuite son ascension jusqu'à la surface en réalisant le cycle programmé de collecte de données (Fig. 4g). Après arrivée en surface, détectée par les capteurs, la transmission radioélectrique des données enregistrées commence (Fig. 4h).

La préparation et le montage au laboratoire et en atelier du dispositif suivant l'invention s'opère de la manière suivante : (1) on remplit la capsule 8 au laboratoire et dans une atmosphère inerte et stérile, avec le liquide de référence 9, le bouchon 10 étant en place, (2) on met en place le joint torique 5 et la tige de liaison 3 ; on exerce une pression sur le joint pour assurer son étanchéité soit par un outillage ad hoc, soit en mettant en dépression l'intérieur de la capsule 8. Cette dépression peut elle-même être obtenue soit par pompage de vide à travers un perçage longitudinal (non représenté) de la tige de liaison 3 et fermé par une vis-pointeau (non représentée) soit pas effet ventouse (chauffage puis refroidissement du liquide contenu dans la capsule 8, soit enfin par montage sous vide de l'ensemble ; la solution retenue dépend du compromis qualité/coût répondant au type d'application recherché. Les capteurs 4 étant ainsi protégés, on peut stocker capteurs et capsule 8. En préparant la campagne de mouillage de la station, on monte la tige de liaison 3 sur la demi-coquille percée du corps de sonde, en assurant l'étanchéité par joint et serrage vis-écrou, puis on monte les moyens électroniques 2 et on ferme le corps de sonde. On termine enfin par le montage du harnais décrit ci-dessus, destiné à protéger l'ensemble sonde-capsule des chocs de manutention et de mise à l'eau.

Le procédé et le dispositif selon l'invention permettent de réaliser une base générant des profils de mesures des grandeurs recherchées en un lieu donné de l'océan, ou encore un réseau de bases d'observation spatio-temporelle in situ sur une zone donnée de l'océan, susceptibles de fonctionner pendant plusieurs années à un niveau de performance très élevé, dans des conditions de mise en oeuvre satisfaisant les besoins opérationnels, pour un coût acceptable, qui est et restera en rapide décroissance au cours des prochaines années.

Ces résultats sont obtenus (1) en s'affranchissant de toute utilisation de câbles, de mécanismes de contrôle d'immersion ou de nettoyage de capteurs, coûteux, délicats à mettre en oeuvre à la mer et de durée de vie brève, (2) grâce à la conception de l'ensemble sonde-capsule 8 de protection et de confinement et à la séquence de retournement de sonde du procédé suivant l'invention.

Cette conception permet en effet de réaliser, grâce à un matériel particulièrement simple (trois pièces principales), les fonctions suivantes : (1) pendant le parcage au fond, - la tenue mécanique sous pression de longue durée du corps de sonde, sa forme sphérique (ou ovoïde) permettant de minimiser ses dimensions, son poids et son coût en utilisant des matériaux travaillant seulement en compression, particulièrement bon marché, comme le verre - la protection des capteurs et leur confinement dans un liquide de référence à la pression atmosphérique à haut degré de protection contre la contamination, - le maintien hors pression des connecteurs électriques reliant capteurs et pied d'antenne à l'électronique de la sonde, ce qui permet de réduire considérablement leur coût, (2) au largage de la sonde, une séparation obtenue par un seul mécanisme très simple et très fiable, (3) au retournement, la possibilité de décharger les salissures tombées sur la sonde pendant le parcage, (4) à la remontée, une disposition des capteurs "en avant", permettant leur rinçage par le déplacement de la sonde sans effet de sillage, - une stabilité dynamique et un contrôle de Cx excellents, garanties d'une vitesse de remontée régulière permettant de reconstituer la trajectoire de la sonde et de compléter ou même de supprimer les capteurs de pression, (5) en surface, un comportement dynamique du corps de sonde caractérisé par de faibles mouvements de roulis/tangage et un bouchonnement facile, assurant de bonnes conditions de transmission radioélectrique, l'antenne étant hors d'eau, au-dessus de la ligne de flottaison de la sonde, même dans les mers difficiles, pendant la plupart du temps.

Bien entendu, l'invention n'est pas limitée au procédé et au dispositif décrits ci-dessus qui n'ont été donnés qu'à titre d'exemple. C'est ainsi que la restitution de profondeur pourrait être obtenue par intégration d'un signal délivré par un loch monté sur le corps de sonde.

## Revendications

1. Procédé pour réaliser des profils verticaux de la mesure d'au moins une grandeur caractéristique d'une masse d'eau marine, à l'aide d'une sonde (1-10) plus légère que l'eau et équipée d'un capteur de mesure (4) de ladite grandeur, du type suivant lequel, a) on coule ladite sonde (1-10) sur le fond marin par lestage, b) on commande la remontée de la sonde (1-10) et l'activation du capteur (4) pour l'enregistrement de la mesure de ladite grandeur au long de la colonne d'eau traversée par la sonde (1-10) jusqu'à la surface de la masse d'eau, et c) on commande une transmission des mesures relevées vers un centre d'exploitation de ces mesures,
**caractérisé en ce que**, à l'étape a), on coule la sonde (1-10) avec le capteur (4) disposé du côté d'une extrémité de celle-ci qui est orientée vers le fond marin, et **en ce que**, à l'étape b), on commande un basculement de la sonde (1-10), à la remontée de celle-ci, de manière que ledit capteur (4) soit alors tourné vers la surface de l'eau.

2. Procédé conforme à la revendication 1, **caractérisé en ce qu'**on commande ledit basculement de la sonde (1-10) en dégageant de celle-ci une capsule (8) de protection et de confinement du capteur (4).

3. Procédé conforme à la revendication 2, **caractérisé en ce qu'**on retient ladite capsule (8) de protection sur le fond marin par ledit lestage.

4. Dispositif pour la mise en oeuvre du procédé conforme à la revendication 1, comprenant une sonde (1-10) plus légère que l'eau et équipée d'au moins un capteur de mesure (4) d'une grandeur caractéristique d'une masse d'eau marine, d'une capsule (8) de protection et de confinement dudit capteur (4) et d'un lest (16) raccordé à ladite capsule (8), **caractérisé en ce que** ladite capsule (8) prend la forme d'une enceinte étanche résistante à la pression hydrostatique, la paroi de ladite enceinte étant équipée d'un bouchon (10) à ouverture commandée électriquement pour obtenir la séparation de la capsule (8) et de la sonde (1-10) et la remontée subséquente de celle-ci sous l'effet de la poussée d'Archimède qu'elle reçoit.

5. Dispositif conforme à la revendication 4, **caractérisé en ce qu'**un liquide de référence (9) contenu dans ladite capsule (8) baigne ledit capteur (4) avant la séparation dudit corps et de ladite sonde.

6. Dispositif conforme à la revendication 4 ou 5, **caractérisé en ce qu'**il comprend un harnais (12,13,14) pour retenir temporairement la sonde (1-10) et la capsule (8) en position d'assemblage, et des moyens (17) pour expulser ledit harnais (12,13,14) quand la sonde (1-10) atteint, lors de sa coulée, une profondeur prédéterminée.

7. Dispositif conforme à l'une quelconque des revendications 4 à 6, **caractérisé en ce que** la capsule (8) intègre ledit lest.

8. Sonde formant partie du dispositif conforme à l'une quelconque des revendications 4 à 7, comprenant au moins un capteur (4) d'une grandeur caractéristique d'une masse d'eau marine, des moyens électroniques (2) de traitement d'un signal émis par ledit capteur (4), une antenne (7) alimentée par lesdits moyens pour émettre des signaux radioélectriques représentatifs dudit signal, et des moyens propres à donner une flottabilité positive à ladite sonde, **caractérisée en ce que** ledit capteur (4) est porté par un organe allongé (3) traversant un corps (1) creux rigide constituant à la fois lesdits moyens de flottabilité et un moyen de support dudit organe, ledit capteur (4) étant extérieur audit corps creux alors que lesdits moyens électroniques (2) sont intérieurs à ce corps.

9. Sonde conforme à la revendication 8, **caractérisée en ce que** ledit organe allongé (3) est monté sur ledit corps (1) creux par des moyens de fixation équipés de joints d'étanchéité (5,21) protégeant de la pression hydrostatique des fils de connexion électriques traversant ledit corps (1) pour relier ledit capteur (4) et lesdits moyens électroniques (2).

10. Sonde conforme à la revendication 9, **caractérisée en ce que** ledit organe allongé (3) est conçu pour recevoir une capsule (8) de protection rigide dudit capteur, des moyens d'étanchéité (5') coopérant avec ladite capsule (8) pour protéger ledit capteur (4) et lesdits fils de la pression hydrostatique, jusqu'à la remontée de la sonde.

11. Sonde conforme à l'une quelconque des revendications 8 à 10, **caractérisée en ce que** son centre de gravité et son centre de poussée sont agencés l'un par rapport à l'autre de manière à assurer la remontée de la sonde, à partir d'un fond marin, avec ledit capteur (4) en avant dudit corps creux (1).

12. Sonde conforme à la revendication 11, **caractérisée en ce que** l'antenne (7) est disposée dans ledit corps creux (1) de manière à se trouver sensiblement entièrement au-dessus de la ligne de flottaison de ladite sonde.

13. Sonde conforme à l'une quelconque des revendications 8 à 12, **caractérisée en ce que** ledit corps creux (1) est de forme généralement sphérique.

## Patentansprüche

1. Verfahren zur Erzeugung von vertikalen Messprofilen wenigstens einer meerwassercharakteristischen Größe mittels einer Sonde (1-10), die leichter als Wasser ist und die mit einem Messwertaufnehmer (4) für die genannte Größe versehen ist, wobei verfahrensgemäß die Sonde (1-10) a) durch Beladen mit Ballast auf den Meeresboden abgesenkt wird, b) der Wiederaufstieg der Sonde (1-10) bewirkt und der Messwertaufnehmer (4) aktiviert wird, um die Messung der genannten Größe entlang der durch die Sonde (1-10) bis zu der Oberfläche des Wassers durchquerten Wassersäule zu registrieren und c) eine Übertragung der gewonnenen Messwerte zu einem Auswertungszentrum für die Messwerte bewirkt wird,
**dadurch gekennzeichnet, dass** während des Verfahrensschrittes a) diese Sonde (1-10) derart abgesenkt wird, dass sich der Messwertaufnehmer (4) auf der Seite des Endes dieser Sonde befindet, welches dem Meeresboden zugekehrt ist und dass während des Verfahrensschrittes b) ein Umkippen der Sonde (1-10) beim Wiederaufstieg bewirkt wird, so dass der genannte Messwertaufnehmer (4) nunmehr der Oberfläche des Wassers zugekehrt ist.

2. Verfahren nach Anspruch 1, **dadurch gekennzeichnet, dass** das Umkippen der Sonde (1-10) dadurch bewirkt wird, dass von dieser eine Schutz- und Sicherheitskapsel (8) des Messwertaufnehmers (4) gelöst wird.

3. Verfahren nach Anspruch 2, **dadurch gekennzeichnet, dass** die genannte Schutzkapsel (8) durch Beladen mit Ballast auf dem Meeresboden zurückgehalten wird.

4. Vorrichtung zur Durchführung des Verfahrens gemäß Anspruch 1 mit einer Sonde (1-10), die leichter als Wasser ist und die mit wenigstens einem Messwertaufnehmer (4) für eine meerwassercharakteristische Größe, mit einer Schutz- und Sicherheitskapsel (8) für den Messwertaufnehmer (4) und einem Ballast (16) versehen ist, der mit der Kapsel (8) in Verbindung steht, **dadurch gekennzeichnet, dass** die genannte Kapsel (8) die Form eines dichten, dem hydrostatischen Druck widerstehenden Behälters aufweist, wobei die Wandung des Behälters mit einem elektrisch zu bestätigenden Stopfen (10) versehen ist, um die Trennung der Kapsel (8) und der Sonde (1-10) sowie den Wiederaufstieg Letzterer aufgrund des auf diese einwirkenden Auftriebs zu erreichen.

5. Vorrichtung nach Anspruch 4, **dadurch gekennzeichnet, dass** der genannte Messwertaufnehmer (4) vor der Trennung der genannten Kapsel und der genannten Sonde in eine Bezugsflüssigkeit (9) eintaucht, die in der Kapsel (8) enthalten ist.

6. Vorrichtung nach Anspruch 4 oder 5, **gekennzeichnet durch** ein Geschirr (12,13,14), welches dazu bestimmt ist, die Sonde (1-10) und die Kapsel (8) vorrübergehend in ihrer Montageposition zu halten und **durch** Mittel (17), um das Geschirr (12,13,14) abzustoßen, sobald während des Absenkens die Sonde (1-10) eine vorherbestimmte Tiefe erreicht hat.

7. Vorrichtung nach einem der Ansprüche 4 bis 6, **dadurch gekennzeichnet, dass** die Kapsel (8) mit dem Ballast verbunden ist.

8. Sonde als Teil der Vorrichtung nach einem der Ansprüche 4 bis 7, bestehend zumindest aus einem Messwertaufnehmer (4) für eine meerwassercharakteristische Größe, elektronischen Mitteln (2) zur Behandlung eines durch den Messwertaufnehmer (4) ausgestrahlten Signals, einer Antenne (7), die durch die genannten Mittel gespeist wird und die zur Ausstrahlung von Funksignalen, die das genannte Signal darstellen, eingerichtet ist und aus Mitteln, die geeignet sind, der genannten Sonde eine positive Schwimmfähigkeit zu geben, **dadurch gekennzeichnet, dass** der genannte Messwertaufnehmer (4) durch ein langgestrecktes Teil (3) getragen ist, welches einen starren Hohlkörper (1) durchdringt, welcher gleichzeitig die genannten, die Schwimmfähigkeit begründenden Mittel und ein Mittel zum Stützen des genannten Teils bildet, wobei sich der Messwertaufnehmer (4) auf der Außenseite und die genannten elektronischen Mittel (2) auf der Innenseite des genannten Körpers befinden.

9. Sonde nach Anspruch 8, **dadurch gekennzeichnet, dass** das genannte langgestreckte Teil (3) an dem genannten Hohlkörper (1) mittels solcher Befestigungsmittel angebracht ist, die mit Dichtungen (5,21) versehen sind, durch welche die, durch den genannten Körper (1) hindurchgeführten, den Messwertaufnehmer (4) mit den genannten elektronischen Mitteln (2) verbindenden elektrischen Verbindungsleitungen vor dem hydrostatischen Druck geschützt sind.

10. Sonde nach Anspruch 9, **dadurch gekennzeichnet, dass** das genannte langgestreckte Teil dazu bestimmt ist, eine starre Schutzkapsel (8) für den genannten Messwertaufnehmer und Mittel zur Abdichtung (5') aufzunehmen, die mit der genannten Kapsel (8) zusammenwirken, um den Messwertaufnehmer (4) und die genannten Leitungen bis zum Wiederaufstieg der Sonde vor dem hydrostatischen Druck zu schützen.

11. Sonde nach einem der Ansprüche 8 bis 10, **dadurch gekennzeichnet, dass** der Schwerpunkt der Sonde und der Mittelpunkt der durch sie verdrängten Flüssigkeit dahingehend relativ zueinander angeordnet sind, dass sichergestellt ist, dass der Wiederaufstieg der Sonde ausgehend von dem Meeresboden so erfolgt, dass sich der genannte Messwertaufnehmer (4) auf der Oberseite des genannten Hohlkörpers (1) befindet.

12. Sonde nach Anspruch 11, **dadurch gekennzeichnet, dass** die Antenne (7) an dem genannten Hohlkörper (1) so angebracht ist, dass sie sich deutlich und vollständig oberhalb der Wasserlinie der genannten Sonde befindet.

13. Sonde nach einem der Ansprüche 8 bis 12, **dadurch gekennzeichnet, dass** der genannte Hohlkörper eine im Wesentlichen kugelförmige Gestalt aufweist.

## Claims

1. A method of producing vertical profiles of the measured value of at least one variable characteristic of a body of sea water using a lighter-than-water probe (1-10) equipped with a sensor (4) for measuring said variable, of the kind in which: a) said probe (1-10) is sunk to the sea bed by a ballast weight, b) ascent of the probe (1-10) and activation of the sensor (4) are commanded to record the measured value of said variable along the column of water along which the probe passes (1-10) to the surface of the body of water, and c) transmission of the measured values obtained to a center for processing those measured values is commanded,
**characterized in that**, in step a), the probe (1-10) is sunk with the sensor (4) on a side thereof facing toward the sea bed and **in that**, in step b), overturning of the probe (1-10) is commanded, during its ascent, so that said sensor (4) is then facing towards the surface of the water.

2. A method according to claim 1, **characterized in that** said overturning of the probe (1-10) is commanded by releasing therefrom a capsule (8) which protects and confines the sensor (4).

3. A method according to claim 2, **characterized in that** said protection capsule (8) is retained on the sea bed by said ballast weight.

4. A device for implementing the method according to claim 1, including a lighter-than-water probe (1-10) equipped with at least one sensor (4) for measuring a variable characteristic of a body of sea water, a capsule (8) for protecting and confining said sensor (4) and a ballast weight (16) connected to said capsule (8), **characterized in that** said capsule (8) takes the form of a watertight enclosure resistant to hydrostatic pressure, the wall of said enclosure being equipped with a plug (10), opening of which is commanded electrically to bring about separation of the capsule (8) and the probe (1-10) and the subsequent ascent thereof due to the effect of the Archimedian upthrust thereon.

5. A device according to claim 4, **characterized in that** said sensor (4) is immersed in a reference liquid (9) contained in said capsule (8) before the separation of said body and said probe.

6. A device according to claim 4 or 5, **characterized in that** it includes a harness (12, 13, 14) for temporarily retaining the probe (1-10) and the capsule (8) in an assembled position and means (17) for expelling said harness (12, 13, 14) when the probe (1-10) reaches a predetermined depth as it sinks.

7. A device according to any of claims 4 to 6, **characterized in that** the capsule (8) integrates said ballast weight.

8. A probe forming part of a device according to any of claims 4 to 7, including at least one sensor (4) of a variable characteristic of a body of sea water, electronic means (2) for processing a signal emitted by said sensor (4), an antenna (7) fed by said means for transmitting radio signals representative of said signal, and means adapted to impart positive buoyancy to said probe, **characterized in that** said sensor (4) is carried by an elongate member (3) passing through a rigid hollow body (1) constituting said buoyancy means and support means for said member, said sensor (4) being outside said hollow body and said electronic means (2) being inside said body.

9. A probe according to claim 8, **characterized in that** said elongate member (3) is mounted on said hollow body (1) by fixing means equipped with seals (5, 21) protecting from hydrostatic pressure electrical connecting wires passing through said body (1) to connect said sensor (4) and said electronic means (2).

10. A probe according to claim 9, **characterized in that** said elongate member (3) is designed to receive a rigid capsule (8) for protecting said sensor, sealing means (5') cooperating with said capsule (8) to protect said sensor (4) and said wires from the hydrostatic pressure until the probe ascends.

11. A probe according to any of claims 8 to 10, **characterized in that** its center of mass and its center of upthrust are arranged relative to each other so that the probe ascends from the sea bed with said sensor (4) at the front of said hollow body (1).

12. A probe according to claim 11, **characterized in that** the antenna (7) is disposed in said hollow body (1) so that it is substantially entirely above the waterline of said probe.

13. A probe according to any of claims 8 to 12, **characterized in that** said hollow body (1) is of generally spherical shape.
